# EUROPÄISCHE PATENTANMELDUNG

(11) **EP 2 746 773 A1**
(43) Veröffentlichungstag der Anmeldung: **25.06.2014**
(21) Anmeldenummer: 13198264.7
(22) Anmeldetag: 19.12.2013
(51) Int. Cl.: G01N 33/569

(54) **Verfahren zur Bestimmung des Grades der allgemeinen Immunsuppression in einem Individuum und Anwendung hiervon**

(30) Priorität: 19.12.2012 DE 102012112600
(71) Anmelder: Medizinische Hochschule Hannover, 30625 Hannover (DE)
(72) Erfinder: Pape, Lars, Prof. Dr. med., 30655 Hannover (DE); Ahlenstiel, Thurid, Dr. med., 30659 Hannover (DE)
(74) Vertreter: Kröncke, Rolf

(57) **Zusammenfassung**

Die vorliegende Erfindung betrifft in einem ersten Aspekt ein Verfahren zur Bestimmung des Grades der Immunsuppression in einem Individuum, das mit einem immunsupprimierenden Mittel behandelt wurde. Weiterhin wird ein Verfahren zur Stratifizierung der Immunsuppression und des therapeutischen Regimes in einem Individuum, das mit einem immunsupprimierenden Mittel behandelt wurde, wird oder behandelt werden soll, beschrieben. Schließlich wird ein Verfahren zur Überwachung der Immunsuppression in einem Individuum, das mit einem immunsupprimierenden Mittel behandelt wird und/oder wurde, ausgeführt. Die Verfahren beruhen auf der Beobachtung, dass virus-spezifische CD4 T-Zellen nach in vitro-Stimulation mit virus-spezifischen Antigenen in einer Blutprobe des Individuums bestimmt werden und auf Basis hiervon der Grad der Immunsuppression im Allgemeinen bestimmt werden kann. Hierdurch kann eine Immunsuppression insbesondere nach Transplantation gesteuert werden. Weiterhin betrifft die vorliegende Erfindung die Verwendung von Kits oder Assays, die zur Bestimmung Virus-spezifischer T Zellen und damit des Grades der Immunsuppression insbesondere bei solider Organtransplantation geeignet sind.

## Beschreibung

Die vorliegende Erfindung betrifft in einem ersten Aspekt ein Verfahren zur Bestimmung des Grades der allgemeinen Immunsuppression in einem Individuum, das mit einem immunsupprimierenden Mittel behandelt wird oder werden soll. Weiterhin wird ein Verfahren zur Stratifizierung der allgemeinen Immunsuppres-sion und des therapeutischen Regimes in einem Individuum, das mit einem immunsupprimierenden Mittel behandelt wurde, wird oder behandelt werden soll, beschrieben. Schließlich wird ein Verfahren zur Überwachung der allgemeinen Immunsuppression in einem Individuum, das mit einem immunsupprimierenden Mittel behandelt wird und/oder wurde, ausgeführt. Die Verfahren beruhen auf der Beobachtung, dass virus-spezifische CD4 T-Zellen nach Stimulation dieser mit virus-spezifischen Antigenen in einer Blutprobe des Individuums bestimmt werden und auf Basis hiervon der Grad der Immunsuppression im Allgemeinen bestimmt werden kann. Hierdurch kann eine Immunsuppression insbesondere nach Transplantation gesteuert werden. Weiterhin betrifft die vorliegende Erfindung die Verwendung von Kits oder Assays sowie dieser Verfahren, die zur Bestimmung der Immunsuppression insbesondere bei solider Organtransplantation ausgebildet sind.

### Stand der Technik

Immunsupprimierende Mittel werden Personen häufig verabreicht, um eine überschießende Immunantwort zu reduzieren oder um z.B. im Falle von Organtransplantationen eine mögliche Abstoßungsreaktion zu vermeiden. Auf dem Gebiet der Transplantation und insbesondere auf dem Gebiet der soliden Organtransplantation sind immunsupprimierte Therapien Standardanwendungen, um eine Abstoßungsreaktion zu verhindern. Eine große Gefahr und ein großes Problem bei Verabreichung von immunsupprimierenden Mitteln ist allerdings, dass generell die natürliche Balance der Immunantwort gegen intra- und extrazelluläre Bestandteile, wie Viren oder Bakterien, gestört wird. Eine nach einer Organtransplantation übliche immunsupprimierte Therapie birgt insbesondere die Gefahr, ernste Komplikationen durch virale Neuinfektion oder die Reaktivierung bereits vorhandener, latenter Infektionen, wie z.B. mit Cytomegalievirus (CMV), Epstein-Barr-Virus (EBV) oder Polyoma-BK-Virus (BKV) hervorzurufen.

Diese oben genannten Viren sind als wesentliche Pathogene z.B. nach Nierentransplantationen bekannt.

Eine immunsupprimierende Therapie ist mit hohen Kosten verbunden und, wie ausgeführt, mit möglichen schweren Nebenwirkungen. Entsprechend sollte eine solche Therapie nur in dem wirklich notwendigen Maße eingesetzt werden.

Es besteht daher ein großes Interesse daran, eine entsprechende Therapie der generellen Immunsuppression im Individuum derart steuern zu können, dass die Therapie so dosiert wird, dass das Risiko Infektionen zu erleiden möglichst gering ist. Allerdings gibt es derzeit noch kein geeignetes System dieses zu leisten. Ein Monitoring durch Bestimmung der Virenlast oder durch Virenserologie ist in vielen Fällen nicht ausreichend, um das individuelle Risiko und damit die Notwendigkeit und Dauer einer prophylaktischen Behandlung zu ermitteln. Es wurde gezeigt, dass virus-spezifische T-Zellen eine signifikante Rolle bei der Kontrolle der Virusreplikation spielen. Daher wurde über die Detektion virus-spezifischer T-Zellen als prognostischer Marker zum Monitoring viraler Erkrankungen spekuliert. Ein Zusammenhang zwischen der Anzahl virus-spezifischer T-Zellen und viraler Komplikationen wurde in mehreren Studien nachgewiesen, z.B. Sester et al., 2001, Transplantation, 71, 1287-1294 oder Widmann T. et al, 2008, PLoS ONE, 3(11), e3634. Dabei wurde geschlussfolgert, dass verminderte Zahlen von spezifischen T-Zellen gegen das jeweilige Virus in Transplantatempfängern mit vermehrten infektiösen Komplikationen gegenüber diesem Virus korrelieren. Die Untersuchungen wurden häufig mit CMV-spezifischen T-Zellen durchgeführt. Es konnte beobachtet werden, dass CMV-Reaktivierungen nach Nierentransplantationen durch erniedrigte Zahl von T-Zellen einer erhöhten Viruslast vorausgehen.

Ziel ist vorliegend die Bereitstellung von Verfahren zur Bestimmung des Grades der allgemeinen Immunsuppression in einem Individuum, insbesondere bei Individuen, die einer Organtransplantation, wie einer soliden Organtransplantation, insbesondere Nierentransplantation, unterworfen wurden.

Diese Aufgabe wird mit dem erfindungsgemäßen Verfahren und deren Anwendung insbesondere bei der Posttransplantbehandlung erreicht.

### Beschreibung der Anmeldung

Die vorliegende Anmeldung stellt ein Verfahren zur Bestimmung des Grades der Immunsuppression in einem Individuum, das mit einem immunsupprimierenden Mittel behandelt wurde, wird oder behandelt werden soll, bereit. Dieses Verfahren umfasst die Schritte
a) Bestimmen von virus-spezifischen CD4 T-Zellen nach Stimulation dieser mit virus-spezifischen Antigenen in einer Blutprobe des Individuums,
b) Bestimmen des Grades der Immunsuppression des Individuums auf Basis des Niveaus oder der Anzahl an virus-spezifischen CD4 T-Zellen bestimmt in Schritt a).

Vorliegend wird, soweit nicht anders ausgeführt, unter dem Ausdruck "Grad der Immunsuppression in einem Individuum" verstanden, dass der Grad der Immunsuppression im Allgemeinen in dem Individuum gemeint ist. D. h. der Grad der Immunsuppression bezieht sich auf die allgemeine Immunantwort im Individuum. Es zeigte sich vorliegend, dass die virus-spezifischen T-Zellen, gerichtet auf einen bestimmten Virus, als Surrogatmarker für die allgemeine Immunantwort, z. B. gegenüber anderen Viren, als das vorliegend bestimmte, aber auch anderen eine Immunantwort auslösenden Antigenen, geeignet ist.

Weiterhin wird ein Verfahren zur Stratifizierung der Immunsuppression und des therapeutischen Regimes in einem Individuum, das mit einem immunsupprimierenden Mittel behandelt wurde, wird oder behandelt werden soll, bereitgestellt, umfassend die Schritte:
a) Bestimmen von virus-spezifischen CD4 T-Zellen nach Stimulation dieser mit virus-spezifischen Antigenen in einer Blutprobe des Individuums,
b) Bestimmen des Grades der Immunsuppression des Individuums auf Basis des Niveaus oder der Anzahl an virus-spezifischen CD4 T-Zellen bestimmt in Schritt a).
c) Bestimmen der weiteren Dosierung der Immunsuppression und des therapeutischen Regimes in diesem Individuum auf Basis des Grades der Immunsuppression gemäß Schritt b).

Schließlich wird ein Verfahren zur Überwachung der Immunsuppression in einem Individuum, das mit einem immunsupprimierenden Mittel behandelt wird und/oder wurde, bereitgestellt, umfassend die Schritte:
a) Bestimmen von virus-spezifischen CD4 T-Zellen nach Stimulation dieser mit virus-spezifischen Antigenen in einer ersten Blutprobe eines Individuums,
b) Bestimmen von virus-spezifischen CD4 T-Zellen nach Stimulation dieser mit virus-spezifischen Antigene in einer zweiten, zeitlich getrennt von der ersten Blutprobe erhaltenen Blutprobe eines Individuums,
c) Vergleich des Niveaus oder der Anzahl an virus-spezifischen CD4 T-Zellen, bestimmt in Schritt a) mit dem Niveau oder der Menge, bestimmt in Schritt b) oder mit einem Referenzwert, wobei eine Erhöhung des Niveaus oder der Anzahl von virus-spezifischen CD4 T-Zellen für eine Erniedrigung des Grades der Immunsuppression indikativ ist und wobei eine Verringerung des Niveaus oder der Anzahl an virus-spezifischen CD4 T-Zellen indikativ für eine Erhöhung des Grades der Immunsuppression ist.

Vorliegend wird unter dem Ausdruck "umfassend" oder "umfasst" sowie unter dem Ausdruck "enthaltend" oder "enthält" auch die Ausführungsform "bestehend aus" verstanden als eine besondere Ausführungsform hiervon.

Weiterhin wird soweit nicht anders ausgeführt unter dem Ausdruck "bestimmen" verstanden, dass dieses ein physisches identifizieren der Zellen und ein quantitatives oder relatives Erfassen der Zahl der Zellen umfasst.

Es konnte vorliegend zum ersten Mal gezeigt werden, dass zwischen dem Niveau von T-Zellen, gerichtet gegen ein spezifisches Virus im Individuum, und der Suszeptibilität gegenüber Infektionen im Allgemeinen, z. B. andere Viren aber auch Bakterien etc., eine Korrelation besteht.

D.h., es wurde von den Erfindern festgestellt, dass die Bestimmung von virus-spezifischen CD4 T-Zellen nicht nur eine Aussage über die virusspezfische Situation der Individuen bzw. über die zelluläre Antwort gegen das Virus sondern auch die zelluläre Immunantwort im Allgemeinen erlaubt. Das heißt, die Bestimmung der Anzahl an virus-spezifischen CD4 T-Zellen in einer Blutprobe eines Individuums, das mit einem immunsupprimierenden Mittel behandelt wurde, wird oder behandelt werden soll, ist ausreichend, den allgemeinen Zustand der zellulären Immunabwehr des Individuums zu bestimmen und zu bewerten.

Bei abfallender Zahl virus-spezifischer CD4 T-Zellen ist somit nicht nur die Immunabwehr bzw. die Unterdrückung einer möglichen latenten Infektion mit dieser Virusart verschlechtert, sondern die allgemeine zelluläre Immunabwehr ist aufgrund der Immunsupprimierung durch immunsupprimierende Mittel verringert.

Eine solche Überimmunsuppression ist aber gerade im Falle von transplantierten Patienten nicht gewünscht, da die Risiken für Nebenwirkungen deutlich ansteigen. Vielmehr sollte die Immunsuppression nur in einem derartigen Umfang erfolgen, dass keine Abstoßung des Transplantats erfolgt, ohne die allgemeine Immunabwehr zu stark zu schwächen. Es zeigte sich, dass bisherige Verfahren basierend auf Talspiegelmessungen der Immunsuppressiva mit dem erfindungsgemäßen Verfahren ergänzt werden können, um den Immunstatus und den Grad der Immunsuppression in dem Individuum genauer zu bestimmen. Dabei ist nicht nur die Bestimmung des Grades der Immunsuppression im Hinblick auf eine mögliche Infektion mit dem jeweilgen Virus, für das die T-Zellen spezifisch sind, möglich, sondern insgesamt die ungewollte Schwächung der Immunabwehr im Allgemeinen bestimmbar.

Es konnte vorliegend überraschend gefunden werden, dass bei Bestimmung von z.B. CMV-spezifischen CD4 T-Zellen eine Verringerung dieser unter ein bestimmtes Niveau nicht nur eine CMV-Infektion begünstigt, sondern auch Infektionen mit anderen latenten Viren, wie EBV-Infektionen, gleiches gilt umgekehrt.

Die Bestimmung der virus-spezifischen T-Zellen erfolgt üblicherweise aus einer Blutprobe des Patienten. Es ist dabei bevorzugt, dass die virus-spezifischen CD4 T-Zellen mittels Immunoassays bestimmt werden, bevorzugt mittels Durchflusszytometrieanalyse. Hierdurch ist eine durchgehende Überwachung der Immunsuppression auf Basis der Bestimmung der virus-spezifischen CD4 T-Zellen möglich. Zur durchflusszytrometrischen Analyse oder zur Analyse mittels anderer Immunoassays werden die Blutleukozyten üblicherweise mit einem Virusantigen stimuliert. Geeignete Antigene sind übliche bekannte Virusantigene von CMV, ADV, HSV, EBV oder BKV.

Es ist z.B. nicht vorgesehen, dass eine Bestimmung der virus-spezifischen T-Zellen derart erfolgt, dass z. B. CMV-spezifische T-Zellen bestimmt werden, um die Auswirkung hiervon auf die CMV Virämie zu erlauben.

Nach Aktivierung mit einem virus-spezifischen Antigen werden die Zellen dann entsprechend mit bekannten Verfahren nachgewiesen. Es ist dabei bevorzugt, dass das Niveau oder die Anzahl der virus-spezifischen CD4 T-Zellen bestimmt wird als CD69 positive und IFNγ/TNFα-positive CD4 T-Zellen.

Die Blutlymphozyten können im Vollblutassay bestimmt werden. Es ist in einer Ausführungsform bevorzugt, dass costimulatorische Mittel hinzugefügt werden, wie costimulatorische Antikörper, z.B. anti-CD28- und anti-CD49d-Antikörper, um die Stimulierung von aktivierten T-Zellen zu verbessern. Die Aktivierung der T-Zellen führt zu einer Anreicherung von Cytokinen, insbesondere IFNγ/TNFα. Durch entsprechende Mittel, wie z.B. Brefeldin A, kann die Sekretion dieser Zytokine aus den Zellen unterdrückt werden, so dass eine Anreicherung von durch die Zellen nach Aktivierung produzierten Zytokinen in den Zellen selbst erfolgt. Diese so mittels Virusantigen aktivierten T-Zellen können dann aufgrund ihrer Anreicherung von Zytokinen, wie TNFα und IFNγ, in Kombination mit weiteren Markern, insbesondere in Kombination mit CD69 und CD4 als aktivierte virus-spezifische CD4 T-Zellen detektiert werden, insbesondere mit Hilfe eines Durchflusszytometers.

Dem Fachmann sind die geeigneten Kulturbedingungen für die Stimulation der Zellen bekannt. Der Fachmann kann insbesondere einfach die notwendigen Konzentrationen der Stimulantien erhalten, um die virus-spezifischen CD4 T-Zellen zu aktivieren.

Zur Bestimmung der aktivierten T-Lymphozyten werden diese bevorzugt vor dem Markieren mit geeigneten Antikörpern fixiert, z.B. mittels Paraformaldehyd. Anschließend erfolgt die Immunfärbung mit Hilfe geeigneter Antikörper, z.B. Antikörper mit entsprechenden Fluoreszenzlabeln, wie Antikörper gerichtet gegen CD4, CD8, CD69 sowie IFNγ und/oder TNFα.

Mit Hilfe von z.B. einem Durchflusszytometer kann dann die Anzahl an aktivierten fluorezenzmarkierten virus-spezifischen CD4 T-Zellen in der Blutprobe bestimmt werden.

Es zeigte sich dabei, dass bei Abfall dieser virus-spezifischen T-Zellen unter eine Grenze, wie z.B. eine Grenze von zwei Zellen/µl Vollblut, wie von unter einer Zelle µl/Vollblut von einer Überimmunsuppression des Individuums gesprochen werden kann. Hierbei gilt, je stärker die Immunsuppression um so geringer die Anzahl an virus-spezifischen T-Zellen im Vollblut und umgekehrt. Auf Basis dieser Überimmunsuppression besteht die erhöhte Gefahr einer Infektion, z.B. einer Virusinfektion bzw. eines Ausbruchs einer latenten Virusinfektion bzw. ein erhöhtes Risiko anderer Komplikationen aufgrund unzureichender zellulärer Immunabwehr. Ein Monitoring (Überwachen) dieses Niveaus bzw. der Anzahl an virus-spezifischen CD4 T-Zellen im Individuum erlaubt ein Steuern und Überwachen der Behandlung mit immunsupprimierenden Mitteln um einerseits eine unnötige und kostenintensive Behandlung des Patienten zu vermeiden und weiterhin um erhöhte Risiken durch bekannte Nebenwirkungen von immunsupprimierenden Mitteln zu verhindern. Das heißt, die Bestimmung der Zahl an virus-spezifischen CD4 T-Zellen im Blut ist ein geeigneter Indikator (Surrogatmarker) um eine mögliche Überimmunsuppression zu bestimmen, die Immunsuppression in einem Individuum zu überwachen und die Therapie mit immunsupprimierenden Mitteln in einem Individuum zu stratifizieren und zu monitoren. Dieses ist insbesondere im Bereich des Posttransplantationsmanagements wichtig und optimiert die personalisierte Therapie des Individuums, insbesondere des Individuums, das ein Transplantat erhalten hat.

Es zeigte sich, dass das erfindungsgemäße Verfahren insbesondere geeignet ist, den Grad der Immunsuppression eines Individuums bzw. das therapeutische Regime in dem Individuum in dem Individuum zu überwachen bzw. zu bestimmen, wenn diese mit immunsupprimierendem Mitteln behandelt werden. Bei den immunsupprimierenden Mitteln kann es sich um übliche z.B. in der Transplantationsmedizin eingesetzte Mittel handeln, wie Everolimus, Cyclosporin A usw.

Es zeigte sich z.B., dass die Bestimmung der Zellzahl der CMV bzw. ADVspezifischen CD4 T-Zellen im Blut ausreichend ist, den Grad der Immunsuppression im Individuum insgesamt zu bestimmen.

Unter dem Ausdruck "Immunsuppression" wird vorliegend ein Vorgang verstanden, der immunologische Prozesse unterdrückt. Die Immunsuppression schließt dabei insbesondere die Unterdrückung der zellulären Immunabwehr ein.

Virusspezifische CD4 T-Zellen sind somit geeignete Surrogatmarker für die individuelle zelluläre Immunabwehr.

Dieser Surrogatmarker erlaubt insbesondere die Dosierung der immunsupprimierenden Mittel zu steuern und gegebenenfalls frühzeitig Gegenmaßnahmen aufgrund von Überimmunsuppression einzuleiten.

Auf dem Gebiet der Transplantation, wie z.B. der Nierentransplantation aber auch anderer Transplantationen, ist dieses von entscheidender Bedeutung, um einerseits eine Abstoßungsreaktion ausreichend zu unterdrücken und andererseits das Risiko von infektbedingten Komplikationen im immunsupprimierten Individuum zu reduzieren.

In einem weiteren Aspekt richtet sich die vorliegende Erfindung auf die Verwendung der erfindungsgemäßen Verfahren zur Steuerung der Posttransplantationsbehandlung insbesondere bei Individuen, die einer Nierentransplantation unterzogen wurden.

Schließlich stellt die vorliegende Erfindung die Verwendung eines Kits oder Assays bereit. Diese Kits oder Assays sind hergerichtet zur Bestimmung des Grades der Immunsuppression in einem Individuum, das mit einem immunsupprimierenden Mittel behandelt wurde, wird oder behandelt werden soll, zur Stratifizierung der Immunsuppression des therapeutischen Regimes in einem Individuum, das mit einem immunsupprimierenden Mittel behandelt wurde, wird oder behandelt werden soll, zur Bestimmung der Veränderung der Immunsuppression an diesem Individuum, und/oder zur Überwachung der Immunsuppression in einem Individuum. Der Assay oder das Kit umfasst dabei Mittel zur Bestimmung von virus-spezifischen CD4 T-Zellen nach Stimulation dieser mit virus-spezifischen Antigenen. Gegebenenfalls sind diese virus-spezifischen Antigene und optional weitere Mittel zur Kultivierung und Stimulation der Zellen Teil des Kits oder des Assays. Schließlich umfasst das Kit oder der Assay Anweisungen zur Durchführung des Assays oder des Kits gemäß dem erfindungsgemäßen Verfahren. Diese Kits oder Assays sind insbesondere derart ausgebildet, dass sie die durchflusszytometrische Analyse zur Bestimmung von virus-spezifischen CD4 T-Zellen erlaubt. Das heißt, die Assays sind mit entsprechenden Markermolekülen, wie fluoreszenzmarkierten Antikörpern, ausgerüstet, um die durchflusszytometrische Analyse zu ermöglichen.

Der Anmeldegegenstand wird unter Bezugnahme auf die Ausführungsbeispiele weiter erläutert, ohne auf diese beschränkt zu sein.

### Beispiele

Die Methode identifiziert CD4-positive T-Lymphozyten, in denen die Zytokinproduktion durch Virus-Antigene stimuliert wird. Die Analyse von Virus-spezifischen T-Zellen wird in vier Schritten aus heparinisiertem Vollblut durchgeführt.

### Stimulation:

Das Lithium-Heparin Blut sollte vor der Verarbeitung maximal 24h bei 2-8°C gelagert werden. Die Lymphozyten werden in vitro mit Virus-Antigen aktiviert. Dies führt zur Induktion intrazellulärer Zytokin-Produktion. Als Negativkontrolle wird ein Kontroll-Antigen benutzt, das kein virus-spezifisches Antigen enthält. Die Positivkontrolle wird mit Staphylococcus aureus enterotoxin B (SEB) durchgeführt.

### Fixierung:

Die Erythrozyten werden lysiert. Die Lymphozyten werden dann fixiert und gewaschen.

### Immunfärbung:

Aktivierte T-Lymphozyten werden mit Hilfe von fluoreszenzmarkierten Antikörpern gegen CD4, CD69 Interferon γ (IFNγ) und Tumor-Nekrose-Faktor α (TNFα)] gekennzeichnet.

### FACS-Messung:

Mittels Durchfluß-Zytometrie (BD-FACS Calibur / BD-FACS Verse) werden die fluoreszenzmarkierten T-Zellen identifiziert. CD69-positive und IFNy/TNFapositive CD4-T-Lymphozyten repräsentieren die Virus-spezifischen T-Zellen.

### Im Folgenden sind die Schritte detailiert angegeben:

### Stimulation

- Aufbewahrung des Li-Heparinblutes bis 24h im Kühlschrank möglich
- Vorgelegt werden 1µg/ml anti-CD28 und 1µg/ml anti-CD49d 140 µl CD28 und 70 µl CD49d Mischung für vorgelegten Ansatz (im Kühlschrank im Plastikgefäß haltbar)
   davon 1,35 µl Stimulationsansatz pro 450µl Li-Heparin-Blut
   → bzw. 4,05 µl für 3 Proben (Kontrolle, Probe + SEB)
- Benötigtes Blutvolumen zupipettieren, durchmischen und pro Stimulation 450µl Blut auf Röhrchen verteilen
- Vortex
- Pro Stimulationsansatz Antigene zupipettieren (für CMV Antigene und Kontrollantigene jeweils 32µl/ml Vollblut)
   also pro 450 µl für CMV 14,4 µl
   für Kontrolle 14,4 µl
   für SEB 1,125 µl
- Vortex
- 2h Inkubation aufrecht im Brutschrank mit lose angeschraubten Deckeln, 37°C, 6%CO₂
- Zugabe von 10µg/ml Brefeldin A (BFA)
   → bzw. 0,9 µl pro 450 µl
   Vortex
- 4h Inkubation aufrecht im Brutschrank mit lose angeschraubten Deckeln, 37°C, 6%CO₂

### Fixieren der Zellen

- Zugabe von 100µl/ml 20mM EDTA (also 45 µl pro 450 µl)
- 10sec pro Röhrchen vortex
- 15min bei RT inkubieren
- Zugabe von 9ml Becton Dickinson Lysingsolution/ml Vollblut (also 4 ml /450µl)
- Vortex
- 10min inkubieren
- 10min zentrifugieren bei 1300rpm
- Überstand absaugen
- Pellet waschen in 2ml PBS-5%FCS-0.5%BSA-0.07%NaN₃ (FACS Puffer)
- Vortex
- 10min zentrifugieren bei 1300rpm
- Überstand absaugen
- Pellet aufnehmen in 200µl/Probe PBS-5%FCS-0.5%BSA-0.07%NaN₃ (also für 2 Proben 400µl)
- Vortex

Zellen können nun gefärbt werden oder auch über Nacht bei 4°C im Kühlschrank aufbewahrt werden

### FACS Färbung

- Vortex
- Zellen auf FACS Röhrchen verteilen (200 µl/Röhrchen)
- 2ml FACS-Puffer/0.1% Saponin zupipettieren
- 10 min inkubieren, RT (offen)
- 10min zentrifugieren bei 1300rpm
- Überstand absaugen
- Zugabe von AK-Mischung in 50µl Gesamtvolumen/ Probe Antikörper: Anti CD4 APC, Anti CD69 PerCP, Anti IFNγ FITC, Anti TNF alpha FITC + SAP 5% und FACS Puffer
- Vortex
- 30-45 min inkubieren, RT, dunkel (offen)
- 3ml FACS-Puffer (ohne Saponin) zupipettieren
- 10min zentrifugieren bei 1300rpm
- Überstand absaugen
- 200µl 1% PFA zupipettieren
- Vortex

### FACS-Analyse

Die Fluorescenz-markierten Antikörper werden z.B. mittels eines BD FACS Calibur oder BD FACS Verse analysiert. Die Häufigkeit der virus-spezifischen CD4 T-Zellen wird bestimmt durch den Prozentsatz von CD4-T Zellen, die durch Virus-Antigen erfolgreich aktiviert wurden (CD69 positiv) und Zytokine (IFNγ und TNF alpha) produziert haben. Die Absolutzahl der virus-spezifischen T Zellen wird berechnet basierend auf der Zahl der Gesamtlymphozyten im Blut.

### Bestimmung der EBV/CMV/ADV/HSV DNA Kopien

Die EBV/CMV/ADV/HSV Kopien wurden mittels Roche Light Cycler (PCR) bestimmt.

### Ergebnis

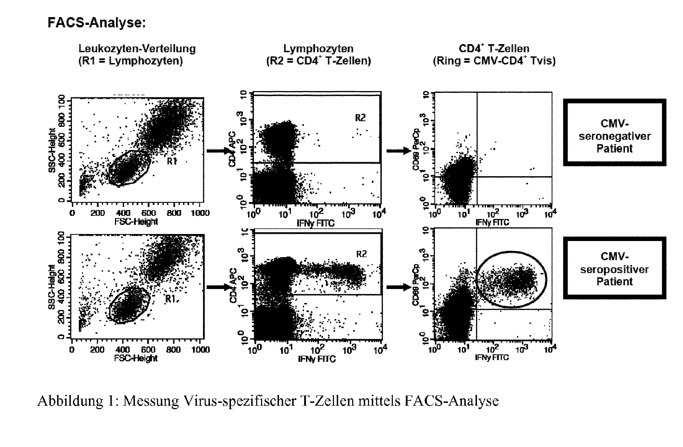

Im Rahmen einer prospektiven Beobachtungsstudie wurde der Verlauf Cytomegalie (CMV)- und Adenovirus (ADV)-Tvis (virus-spezifische T-Zellen) bei 37 Kindern (1-17 Jahre; Median 13 Jahre) im ersten Jahr nach NTx untersucht. Für die Analyse der Tvis wurde das Patientenblut mit CMV- oder ADV-Antigen stimuliert und mit fluoreszenz-markierten Antikörpern gefärbt. Basierend auf spezifischer Zellaktivierung und Induktion intrazellulärer Zytokine erfolgte die Bestimmung CMV- und ADV-CD4+ und CD8+Tvis mittels FACS-Analyse. Zeitgleich wurden Virusinfektionen und Virus-DNA dokumentiert. Ein Beispiel hierfür ist in der Abbildung 1 dargestellt.

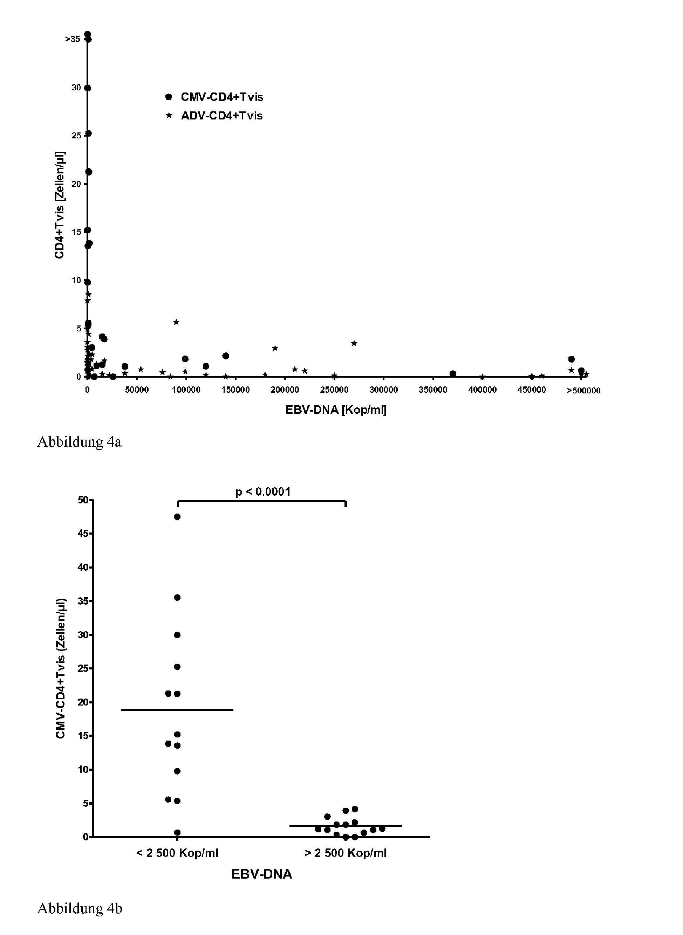

Bei seropositiven Patienten waren CMV- und ADV-CD4+Tvis permanent nachweisbar und schwankten in Abhängigkeit vom Grad der Immunsuppression: Unter der intensivierten Immunsuppression in der Initialphase nach NTx zeigte sich ein vorübergehender Abfall der CD4+Tvis. Nach Reduktion der Immunsuppressiva stiegen die CD4+Tvis wieder an. In Gegenwart einer ausreichenden Zahl von ADV- oder CMV-CD4+Tvis (>2 Zellen/µl) wurden keine symptomatischen Virusinfektionen oder -reaktivierungen beobachtet. Patienten mit niedrigen CD4+Tvis (<2 Zellen/µl) zeigten dagegen eine gesteigerte Anfälligkeiten für verschiedene symptomatische Virusinfektionen (z.B. CMV, EBV): Dementsprechend waren symptomatische CMV-Reaktivierungen durch das vorübergehende Verschwinden von CMV-CD4+Tvis gekennzeichnet in Kombination mit niedrigen ADV-CD4+Tvis bei CMV-DNA-Anstieg. Im Falle asymptomatischer CMV-Reaktivierungen verschwand der schwach positive CMV-DNA-Nachweis spontan in Gegenwart hoher Level von CMV- und ADV-CD4+Tvis (>2 Zellen/µl). Darüber hinaus korrelierte die Abwesenheit von CMV- und ADV-CD4+Tvis (<2 Zellen/µl) auch mit einem erhöhten Risiko für EBV-Infektionen/ -Reaktivierungen mit persistierender EBV-DNA-Last (Spearman r= -0,68 and -0,49, p<0,0001). Im Falle einer hohen EBV-DNA-Last (>2500 Kopien/ml) fanden sich signifikant niedrigere CMV- und ADV-CD4+Tvis als ohne relevanten EBV-DNA-Nachweis (CMV-CD4+Tvis: 1,6 ± 1,3/µl vs. 18,8 ± 13,3/µl; p<0,0001). Im Gegensatz zu CD4+Tvis waren CD8+Tvis nur vorübergehend nachweisbar, siehe Abbildung 4.

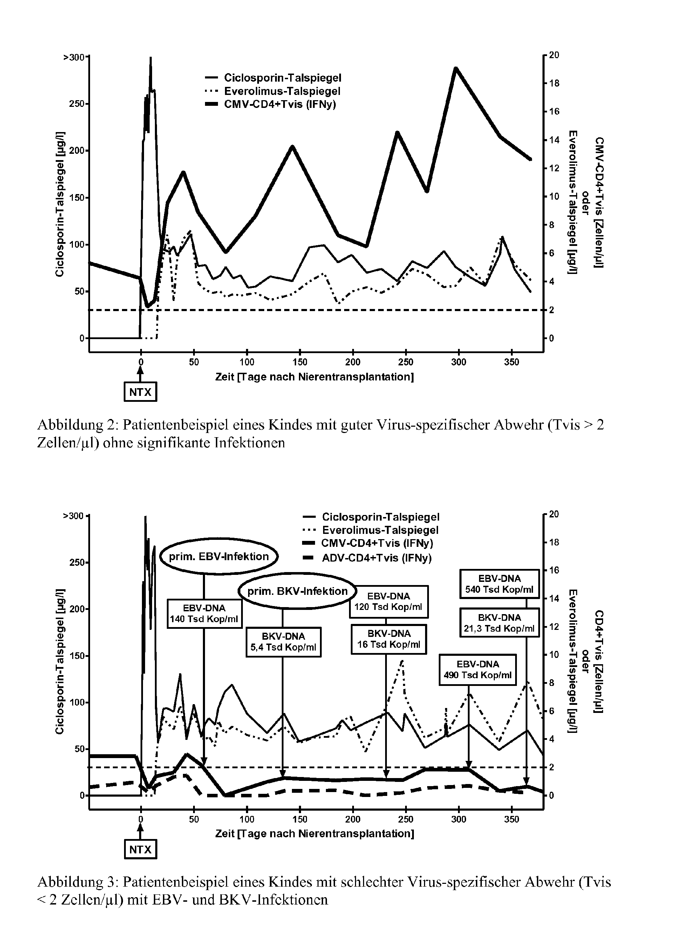

Nach NTx korrelierte die CD4+Tvis-Zahl mit dem individuellen viralen Erkrankungsrisiko, wobei CMV- und ADV-CD4+Tvis nicht nur die virus-spezifische, sondern auch die allgemeine zelluläre Immunabwehr repräsentierten: In Gegenwart einer ausreichenden Zahl von CMV- und ADV-CD4+Tvis (>2 Zellen/µl) fanden sich keine symptomatischen Virusinfektionen, wohingegen ein Abfall (<2 Zellen/µl) mit einem gesteigerten Risiko für virale Komplikationen (v.a. EBV) assoziiert war. Als Marker für "Überimmunsuppression" könnte das Monitoring CMV- und ADV-CD4+Tvis nach NTx dazu beitragen, die Steuerung der immunsuppressiven und antiviralen Therapie zu optimieren (effect-related drugmonitoring), wie in den Abbildungen 2 und 3 dargestellt.

Hieraus wird deutlich, dass virusspezifische T-Zellen nicht nur die virusspezifische sondern auch die allgemeine zelluläre Immunabwehr nach einer Nierentransplantation repräsentiert.

Daraus wird deutlich, dass bei einer ausreichend hohen Anzahl virusspezifischer CD4 T-Zellen, z.B. größer 2 Zellen /µl, keine symptomatischen Virusinfektionen und daraus schlussfolgernd auch eine ausreichende zelluläre Immunabwehr im Allgemeinen vorliegt, während bei einem Absinken der virus-spezifischen CD4 T-Zellen unter ein bestimmtes Niveau, wie kleiner 2 Zellen/µl, ein gesteigertes Risiko für Komplikationen aufgrund zu starker Immunsuppression vorliegt.

Entsprechend ist ein Immunmonitoring von Transplantatempfängern insbesondere bei Nierentransplantationen möglich. Auf Basis der virus-spezifischen CD4 T-Zellen kann die immunsuppressive Therapie gesteuert werden.

## Patentansprüche

1. Verfahren zur Bestimmung des Grades der Immunsuppression in einem Individuum, das mit einem immunsupprimierenden Mittel behandelt wurde, wird oder behandelt werden soll, umfassend die Schritte:
a) Bestimmen von virus-spezifischen CD4 T-Zellen nach in vitro- Stimulation dieser mit virus-spezifischen Antigenen in einer Blutprobe des Individuums,
b) Bestimmen des Grades der Immunsuppression auf Basis des Niveaus oder der Anzahl an virus-spezifischen CD4 T-Zellen bestimmt in Schritt a).

2. Verfahren zur Stratifizierung der Immunsuppression und der Steuerung des therapeutischen Regimes in einem Individuum, das mit einem immunsupprimierenden Mittel behandelt wurde, wird oder behandelt werden soll, umfassend die Schritte:
a) Bestimmen von virus-spezifischen CD4 T-Zellen nach in vitro-Stimulation dieser mit virus-spezifischen Antigenen in einer Blutprobe des Individuums,
b) Bestimmen des Grades der Immunsuppression auf Basis des Niveaus oder der Anzahl an virus-spezifischen CD4 T-Zellen bestimmt in Schritt a).
c) Bestimmen der weiteren Dosierung der weiteren Immunsuppressiven Therapie auf Basis des Grades der Immunsuppression gemäß Schritt b).

3. Verfahren zur Überwachung der Immunsuppression in einem Individuum, das mit einem immunsupprimierenden Mittel behandelt wird und/oder wurde, umfassend die Schritte:
a) Bestimmen von virus-spezifischen CD4 T-Zellen nach in vitro Stimulation dieser mit virus-spezifischen Antigenen in einer ersten Blutprobe eines Individuums,
b) Bestimmen von virus-spezifischen CD4 T-Zellen nach in vitro- Stimulation dieser mit virus-spezifischen Antigenen in einer zweiten, zeitlich getrennt von der ersten Blutprobe erhaltenen Blutprobe eines Individuums,
c) Vergleich des Niveaus oder der Menge an virus-spezifischen CD4 T-Zellen, bestimmt in Schritt a) mit dem Niveau oder der Menge, bestimmt in Schritt b) oder mit einem Referenzwert, wobei eine Erhöhung des Niveaus oder der Anzahl von virus-spezifischen CD4 T-Zellen für eine Erniedrigung der Immunsuppression indikativ ist und wobei eine Verringerung des Niveaus oder der Menge an virus-spezifischen CD4 T-Zellen indikativ für eine verstärkte Immunsuppression ist.

4. Verfahren nach einem der vorherigen Ansprüche, **dadurch gekennzeichnet, dass** die Bestimmung der virus-spezifischen CD4 T-Zellen mittels Immunoassays erfolgt, bevorzugt mittels Durchflusszytometrie-Analyse.

5. Verfahren nach einem der vorherigen Ansprüche, **dadurch gekennzeichnet, dass** die virus-spezifischen CD4 T-Zellen bestimmt werden durch Bestimmung der CD69 positiven und IFNγ/TNFα-positiven CD4 T-Zellen.

6. Verfahren nach einem der vorherigen Ansprüche, wobei die virus-spezifischen CD4 T-Zellen CMV und/oder ADV spezifische CD4 T-Zellen sind.

7. Verfahren nach einem der vorherigen Ansprüche zur Bestimmung von Notwendigkeit, Zeitpunkt und Dauer einer antiviralen Therapie bei dem Individuum.

8. Verfahren nach einem der vorherigen Ansprüche, wobei das Individuum eines ist, das eine immunsuppressive Therapie erhält, insbesondere nach einer Transplantation, wie einer Nierentransplantation.

9. Verfahren nach einem der vorherigen Ansprüche zur Verwendung bei der Steuerung der Immunsuppression nach Transplantation.

10. Verwendung eines Kits oder Assays zur Bestimmung des Grades der Immunsuppression in einem Individuum, das mit einem immunsupprimierenden Mittel behandelt wurde, wird oder behandelt werden soll, zur Steuerung der Immunsuppression und des therapeutischen Regimes in ein Individuum, das mit einem immunsupprimierenden Mittel behandelt wurde, wird oder behandelt werden soll, zur Bestimmung der Veränderung der Immunsuppression an diesem Individuum, und/oder zur Überwachung der Immunsuppression in einem Individuum, dieser Assay oder Kit umfasst Mittel zur Bestimmung von virus-spezifischen CD4 T-Zellen nach *in vitro-*Stimulation dieser mit virus-spezifischen Antigenen, und Anweisungen zur Durchführung des Assays oder Kits gemäß einem Verfahren nach einem der Ansprüche 1 bis 9.

11. Verwendung eines Kits oder Assays nach Anspruch 10, wobei dieser die durchflusszytometrische Analyse und Bestimmung von virus-spezifischen CD4 T-Zellen umfasst.

12. Verwendung des Niveaus oder der Anzahl an virus-spezifischen T-Zellen in einem Individuum als Surrogatmarker für die allgemeine Immunsuppression in diesem Individuum.

13. Verfahren nach einem der Ansprüche 1-9 und 12, wobei bei einer Zellzahl von virus-spezifischen T-Zellen gegen ein spezifisches Virus, insbesondere CMV, von kleiner 2 Zellen/µl Vollblut eine zu starke Immunsuppression vorliegt.

14. Verfahren nach einem der Ansprüche 1 bis 9, 12 oder 13, wobei das Niveau oder die Anzahl an virus-spezifischen T-Zellen gegen ein Virus den Grund der Immunsuppression des Individuums gegenüber anderen Viren bestimmt.
